# EUROPEAN PATENT APPLICATION

(11) **EP 0 841 011 A1**
(43) Date of publication of application: **13.05.1998**
(21) Application number: 97830530.8
(22) Date of filing: 22.10.1997
(51) Int. Cl.: A23L 1/308, A23L 1/30, A23L 1/304, A61K 35/78, A61K 31/375

(54) **Dietary preparation comprising chitosan and other soluble fibres combined with ascorbic acid, organic chromium, vanadium and garcinia hydroxycitrate for lipid absorption lowering and glucide metabolism stabilization**

(30) Priority: 23.10.1996 IT RM960720
(71) Applicant: SIRC S.p.A. NATURAL & DIETETIC FOODS, I-20090 Caleppio Di Settala (MI) (IT)
(72) Inventor: Littera, Renato, 10143 Torino (IT)
(74) Representative: Sarpi, Maurizio

(57) **Abstract**

A preparation based on chitosan and other substances having high fibre content, such as guar flour, is added with an acid, such as ascorbic acid, to increase the effectiveness as fat binding agent and is combined with three proximate principles such as organic chromium, vanadium and garcinia hydroxycitrate so as to synergically act on the stabilization of the glucide and lipid metabolism.

## Description

The present invention relates to a dietary product which is based on chitosan and other soluble fibres in the hypocaloric diets for lowering the body weight and provided with an increased binding activity to the ingested fat together with a particular effectiveness in the stabilization of the sugar metabolism and generally in the hyperinsulinaemia.

More particularly the invention relates to a preparation based on chitosan and other substances having high fibre content such as guar flour added with an acid such as ascorbic acid and three proximate principles such as organic chromium, vanadium and garcinia hydroxycitrate.

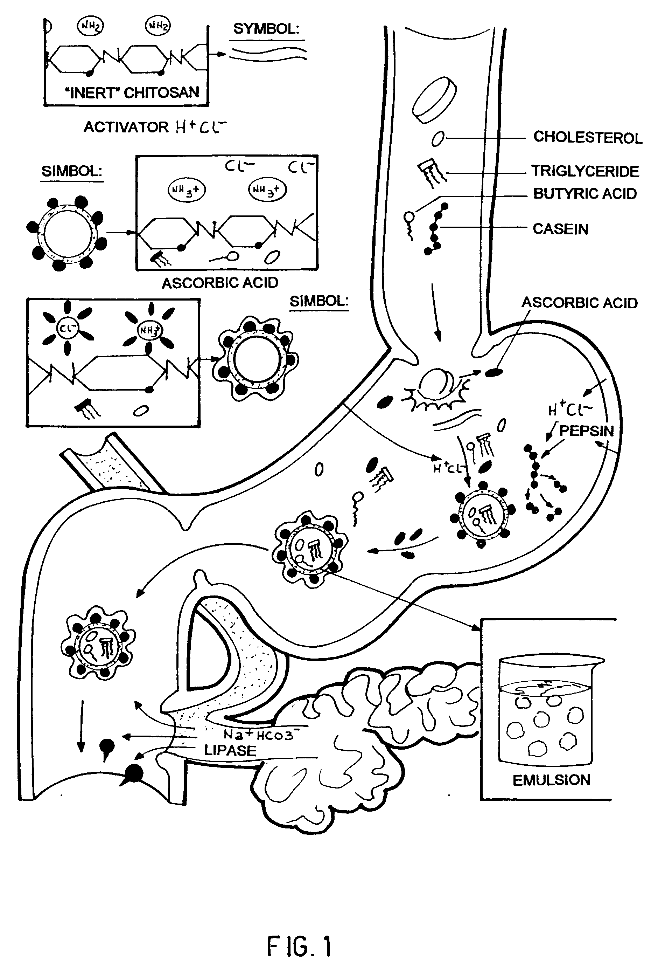

The chitosan binding action to ingested fats is widely known in the technical literature of this field. Recently a marked capability of reducing the calorie amount absorbed by the intestine in relation to the amount ingested with the food was shown by the combination of chitosan provided with electrostatic charge with guar flour, as described in the Italian Patent Application No.RM95A000772 filed on November 24, 1995. Such combination was found, however, to be unable to ensure the same clinical treatment to all of the treated subjects. As chitosan operates by chemical-physical way, the Applicant investigated thoroughly such aspect which could not be understood and supposed that chitosan should dissolve after swallowing and then carry on its activity in the stomach by means of the hydrochloric acid contained in the gastric juice. Actually, the larger the acid environment, the more the -NH₂ groups of chitosan activated to NH₃. Figure 1 shows the action of chitosan. As a result chitosan has a lower activity in subjects having lower amounts of hydrochloric acid in the stomach.

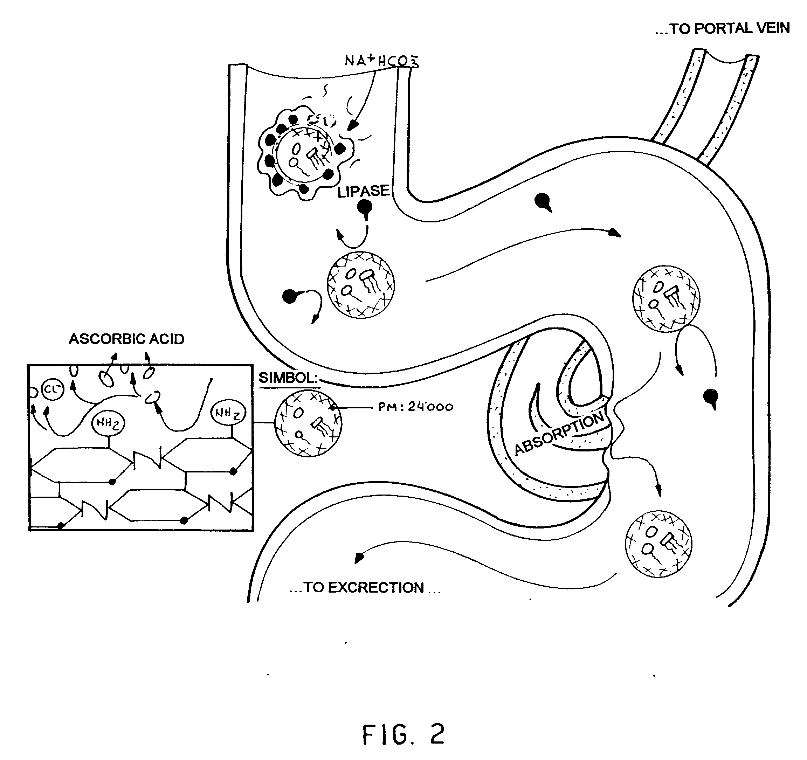

According to such assumption a preparation formed of the combination of chitosan with other soluble fibres, such as guar flour which can be used as dietary integrator or pharmaceutical product, was added with an acid such as ascorbic acid (vitamin C) which, in addition to help the solubilization of chitosan as it is rich in -OH groups, has also a "philia", i.e. tends to form ionic bonds and then encompasses like a "star" the fat particles bound to -NH₃ groups activated by chitosan. When such "star" contacts the basic environment of the intestine, a gelatinous shield is formed which prevents the lipase from attacking the fat acids encompassed by chitosan as well as from splitting them, as shown in Fig. 2.

Trials confirmed all expectations and showed statistically a higher effectiveness of chitosan as binding agent of ingested fats for all of the treated subjects without exceptions.

After having cleared up the activity of chitosan towards fats ingested on a diet, the problem was tackled concerning very traditional diets, such as the Mediterranean diets, where in addition to fats a large amount of carbohydrates is consumed.

The formulation of the dietary preparation was then integrated with three further proximate principles such as vanadium, organic chromium and garcinia hydroxycitrate which typically act on the glucide metabolism.

There is a wide survey in the literature which points out that an integration of 20 micrograms vanadium a day and 100-200 micrograms organic chromium a day are particularly effective in the stabilization of sugar metabolism and generally in the hyperinsulinaemia.

Particularly vanadium buffers glucides because of its activity towards insulin. Such activity is carried on by a phosphorylation of insulin receptors following the activation of tyrosine-kinase contained in the receptors and the inhibition of tyrosine-phosphatase responsible for the dephosphorylation of the insulin receptors.

As for chromium, there is a number of scientific tests that prove the importance of such micronutrient in the human feeding and particularly in the glucide metabolism control. In order to carry on its physiological activity the organic trivalent chromium should be converted into an organic low-molecular-weight complex, the so-called GTF (Glucose Tolerance Factor) . Such complex is a co-factor of insulin capable of increasing the receptor activity thereof by catalyzing the formation of disulfide bridges between the insulin hormone and the sulfhydryl radicals of the receptor with the result of an improvement of the glucide tolerance and the reduction of glycaemia.

The third proximate principle, garcinia hydroxycitrate, inhibits the production of carbohydrate fats. Once swallowed carbohydrates are digested and transformed into glucose. The glucose molecules which are not immediately used to produce energy are stored in liver and muscles in the form of glycogen. When the glycogen supplies are saturated, the exceeding glucose molecules are further divided and converted into fat and cholesterol by an enzyme so-called citratelyase. The hydroxycitrate operates temporarily as inhibitor of such enzyme. The study conducted on animals proved that the hydroxycitrate reduces the synthesis of the fat acid by about 40-70% till eight-twelve hours after meals with a result of a decrease by 27-30% of the production of endogenous cholesterol.

Starting from such scientific tests and following clinical trials a new combination of purified chitosan and other substances having high fibre content, such as guar flour, with ascorbic acid, chromium, vanadium and garcinia hydroxycitrate was found to act synergically and in a surprisingly effective manner on the stabilization of the lipid and glucide metabolism. The proposed percent formulation is as follows:

| | |
|---|---|
| 45-70% | chitosan |
| 8-16% | guar fibre |
| 2-25% | vitamin C |
| 16-32% | garcinia hydroxycitrate |
| 0.200-0.300% | vanadium pentoxide |
| 0.005-0.020% | organic chromium |
| 0.800-1.00% | zinc sulphate |
| 0.750-0.950% | iron lactate |

Such formulation illustrates the present invention without any limitation.

A particularly preferred formulation is as follows:

| | |
|---|---|
| 59.5% | purified chitosan |
| 23.5% | garcinia hydroxycitrate |
| 11.5% | guar fibre |
| 3.6% | vitamin C |
| 0.850% | iron |
| 0.900% | zinc sulphate |
| 0.240% | vanadium |
| 0.007% | organic chromium |

A dose unit was determined in the form of a capsule of 480 mg where the amount by weight of chitosan and other fibres is sufficient for an average daily exhibition with a posology of 2-3 capsules before the main meals or twice a day in weight controlling diets. In keeping diets as well as during hypercholesterolaemia and hyperglycaemia control the posology is decreased to 1 capsule before the main meals or twice a day.

The capsule formulation is as follows:

| | |
|---|---|
| 250 mg | purified chitosan (95% deacetylate) |
| 99 mg | garcinia hydroxycitrate |
| 48 mg | guar fibre |
| 15 mg | vitamin C |
| 3,5 mg | iron |
| 3,75 mg | zinc sulphate |
| 1 mg | vanadium |
| 30 micrograms | organic chromium |

plus excipients quantum sufficit.

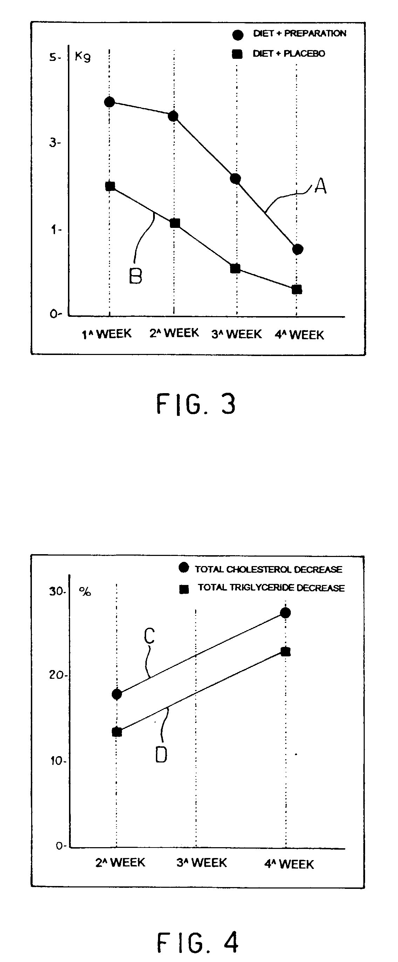

The overweight decrease in the first four weeks of treatment is shown by the diagrams of Fig. 3 where the clinical results of a random double-blind controlled study are compared with a placebo by using two parallel groups of 50 patients. 50 patients were subjected to a hypocaloric diet and the exhibition of 2 capsules of the dietary integrator of the present invention twice a day and 50 patients were subjected to a hypocaloric diet and the exhibition of 2 capsules of placebo twice a day.

As can be seen, the overweight decrease in patients treated with the integrator according to the invention (diagram A) is double as high as those treated with placebo (diagram B).

As for the cholesterol and triglyceride decrease, the effectiveness of the integrator of the present invention is shown by diagrams C and D of Fig. 4, respectively.

The above-mentioned data confirm that the use of a dietary product based on the combination of chitosan and guar flour with vitamin C, garcinia hydroxycitrate, chromium and vanadium can be advantageously prescribed in all of the overweight and obesity dietotherapies as well as in all of the hypercholesterolaemia and hyperglycaemia control diets.

## Claims

1. Use of vitamin C in combination with chitosan in dietary preparations useful for the body weight decrease as well as the hypercholesterolaemia and hyperglycaemia control.

2. A dietary preparation for the overweight and obesity treatment having hypercholesterolaemia and hyperglycaemia control activity, comprising chitosan in combination with vitamin C, garcinia hydroxycitrate, organic chromium and vanadium.

3. The dietary preparation of the preceding claim, characterized in that the dose units of the proximate components are expressed by the following weight percentages:
| | |
|---|---|
| 45-70% | chitosan |
| 8-16% | guar fibre |
| 2-5% | vitamin C |
| 16-32% | garcinia hydroxycitrate |
| 0.200-0.300% | vanadium pentoxide |
| 0.005-0.020% | organic chromium. |

4. The dietary preparation of claims 2 and 3, wherein it also includes:
| | |
|---|---|
| 0.800-1.00% | zinc sulphate |
| 0.750-0.950% | iron lactate. |

5. The dietary preparation of claims 2 to 4, wherein it includes:
| | |
|---|---|
| 59.5% | purified chitosan |
| 23.5% | garcinia hydroxycitrate |
| 11.5% | guar fibre |
| 3.6% | vitamin C |
| 0.850% | iron |
| 0.900% | zinc sulphate |
| 0.240% | vanadium |
| 0.007% | organic chromium |

6. The dietary preparation of claims 2 to 5, characterized in that the dose unit is a capsule including:
| | |
|---|---|
| 250 mg | purified chitosan (95% deacetylate) |
| 99 mg | garcinia hydroxycitrate |
| 48 mg | guar fibre |
| 15 mg | vitamin C |
| 3,5 mg | iron |
| 3,75 mg | zinc sulphate |
| 1 mg | vanadium |
| 30 micrograms | organic chromium |
plus excipients quantum sufficit.
